Europäisches Patentamt

European Patent Office

Office européen des brevets

(11) Publication number: **0 111 144**
**B1**

(12) **EUROPEAN PATENT SPECIFICATION**

(45) Date of publication of patent specification: **03.12.86**

(51) Int. Cl.⁴: **A 61 K 9/24, A 61 K 9/22**

(21) Application number: **83110874.1**

(22) Date of filing: **31.10.83**

(54) Sustained release solid dosage forms having non-uniform distribution of active ingredient.

(30) Priority: **01.11.82 US 438100**
**27.09.83 US 536128**

(43) Date of publication of application:
**20.06.84 Bulletin 84/25**

(45) Publication of the grant of the patent:
**03.12.86 Bulletin 86/49**

(84) Designated Contracting States:
**AT BE CH DE FR GB IT LI LU NL SE**

(56) References cited:
**FR-A-2 331 375**
**FR-A-2 382 235**
**GB-A-1 139 869**
**GB-A-2 042 888**
**US-A-3 965 255**
**US-A-4 292 302**

**THE MERCK INDEX, 9th edition, 1976, Merck &
Co., USA**
**UNLISTED DRUGS, vol. 33, no. 9, September
1981, page 148**

(73) Proprietor: **MERRELL DOW
PHARMACEUTICALS INC.**
**2110 E. Galbraith Road
Cincinnati Ohio 45215 (US)**

(72) Inventor: **Zoglio, Michael A.
6835 Stonington Road
Cincinnati Ohio 45230 (US)**

(74) Representative: **Vossius & Partner
Siebertstrasse 4 P.O. Box 86 07 67
D-8000 München 86 (DE)**

Courier Press, Leamington Spa, England.

## Description

The desirability of sustained-action pharmaceutical forms has long been recognized as desirable in terms of patient convenience and optimum availability of drug. Sustained-action dosage forms provide rapid availability and maintenance of medicament at a level sufficient to provide a desired biological effect. The sustained-action dosage forms are to be distinguished from prolonged-action dosage forms which provide an initial amount of medicament which may exceed the upper limit of the desirable therapeutic range and which subsequently will provide medicament within the desirable therapeutic range for a longer time than will a conventional dosage form. Currently available, so called sustained-action dosage forms operate as prolonged-action dosages and merely mimic the desired sustained-release effect. L. Lachman, H. A. Lieberman and J. L. Kanig, *The Theory and Practice of Industrial Pharmacy*, Lea and Febiger, 1970, page 409.

Traditionally, pharmaceutical dosage forms contain an active component distributed uniformly throughout. For sustained-release solid dosage forms where release of drug is primarily by diffusion or erosion, uniform concentration leads to a non-linear, i.e., non-zero order, release profile. In an eroding system, the surface area exposed to dissolution diminishes with time, thereby causing a reduction in drug-release rate. In systems where diffusion is the primary release mechanism, the rate of release is dependent on the gradient of drug concentration between the dosage form and dissolution environment. Depletion of drug from the system causes a reduction in this gradient which slows release rate. Applicant has discovered that non-uniform concentration can be used as a means of countering diffusion or erosion effects. FR—A—2 331 375 describes a method of preparing granules having a continuously decreasing concentration of active component from the center to the surface. The granule is prepared by continuously coating a particle with a composition containing progressive less of the active ingredient. The granules may be used to prepare pharmaceutical tablets and capsules with controlled release of the active component.

Applicant's novel dosage forms provide for zero-order release of active ingredient and are suitable for use in sustained release formulations. The desired release of medicament is accomplished by preparing a solid dosage form having a core and one or more concentric enveloping layers or press coats wherein the concentration of medicament is greatest in the core and diminishes in each subsequent outer layer. A solid dosage form having such a distribution of medicament avoids the non-linear release of prior art solid dosage forms having uniform distribution of active ingredients. As applicant's dosage forms erode, reducing available surface area, the greater drug concentration in the core negates the diminishment of drug-release rate. Where applicant's dosage forms release drug primarily by diffusion, non-uniform distribution eliminates the reduction of gradient inherent in diffusing dosage forms having uniform drug distribution.

Applicant has discovered that pharmaceutical tablets, having a core and one or more enveloping sustained-release layers, the core and sustained-release enveloping layers consisting essentially of a mixture containing medicament, hydrophilic gel and excipients, wherein the concentration of hydrophilic gel in the core and in each sustained-release enveloping layer is essentially constant, provide for a substantially zero-order release of medicament. The amount of medicament in either the core or in any given sustained-release layer is determined by the formula

$$D_L = D_T \times \frac{R_D}{R_T}$$

wherein $D_L$ is the amount of medicament in the core or in any given sustained-release layer, $D_T$ is the total amount of medicament in the sustained-release portion of the tablet, $R_D$ is the differential radius for the core or the enveloping layer for which the amount of required medicament is to be determined and $R_T$ is the radius of the sustained-release portion of the tablet.

Detailed description of the invention

Solid dosage forms, as used herein, include pressed or compressed tablets, molded tablets, pills and extruded suppositories. Compressed tablets are preferred because of ease in manufacturing and consumer acceptance.

Applicant's solid-dosage forms have a central core and one or more concentric enveloping sustained-release layers or press coats. Ideally, there would be an infinite number of sustained-release layers of infinitesimally small thickness. For practical reasons, primarily due to the limitation of presently available tableting machinery, ten (10) or so layers over the core would be the uppermost limit. For example, the Manesty Bicota®, manufactured by Manesty Machines Limited, can produce a triple pressed tablet from three separate granulations and a fourth coat can be pressed on by using a device to recycle the triple pressed tablet. Applicant's preferred solid-dosage form is a tablet having a core and from 1 to 4 enveloping layers. More preferably, the dosage form would have a core and one enveloping layer.

Applicant also contemplates that one or more outer layers may be used to effect a variety of purposes other than sustained release. For example, tablets may be coated to protect the ingredients from the atmosphere, to mask unpleasant taste, to improve appearance, to control the site of action of the drug such as an enteric coating, and to provide a rapidly dissolving outer layer to provide for quick onset of action followed by zero order sustained release. These optional layers may be accomplished by sugar coating, film coating, compression coating and any other method known to the art. In a preferred embodiment, an outer, quickly-dissolving, non-sustained release layer would contain medicament needed for rapid onset of action and several inner, sustained-release layers and core would contain additional amounts of the medicament or would optionally contain a second medicament whose sustained-release action would compliment the effect of the medicament in the outer, non-sustained release layer. For example, a preferred tablet of applicant's invention has a core and 3 enveloping layers wherein the quickly-dissolving, outermost layer contains terfenadine, an antihistamine, and wherein the sustained-release core and inner layers contain pseudoephedrine hydrochloride, a bronchodilator.

The core and the enveloping layers may be any thickness but solid dosage forms in excess of 25.4 mm thick are quite undesirable from the consumer's point of view. Although it is highly desirable to have a multitude of very thin layers, practical limitations render this goal quite difficult to attain. For example, current tableting technology precludes press-coated layers less than about 0.8 mm thick. Extruded solid dosage forms cannot have layers less than about 1 mm thick. Applicant prefers 1 to 4 press coated layers over a central core, wherein the layers are about 2.4 mm thick.

Applicant's novel dosage forms rely on the ability of hydrophilic gels to swell in contact with water and retard the release of medicament. As used herein, a hydrophilic gel is any substance which gels when in contact with water, and swells slowly from the outer surface to the core resulting in diffusion or erosion of the drug from the swollen matrix. Substances having these properties are well known to those skilled in the pharmaceutical arts. Examples of suitable hydrophilic gels for use in the present invention are hydroxypropyl methylcellulose (HPMC), hydroxypropyl cellulose, hydroxymethylcellulose, hydroxyethylcellulose, hydroxypropyl ethylcellulose, methylcellulose, carboxyethylcellulose, carboxymethyl hydroxyethylcellulose, methylcellulose cross-linked with dialdehyde, Carbopol®, polyvinyl alcohol cross-linked with glyoxal, formaldehyde or glutaraldehyde, poly(N-vinyl-2-pyrrolidone), poly(hydroxyalkyl methacrylate), esters of 2-hydroxyethyl methacrylate, acrylates such as Aquakeeps®, N-vinyl lactams, poly acrylic acids such as Goodrite®, poly acrylamides such as Cyanamer®, maleic anhydride plus styrene co-polymer, maleic anhydride plus ethylene propylene, butylene or isobutylene co-polymer, cross-linked polyethylene oxides, polyethylene oxide, indene anhydride polymer, starch graft polymers, poly glucan cross-linked diesters, cross-linked poly acrylamides, and natural gums such as carraghenin, alginate gum, guar gum and carob gum. Moreover, applicant contemplates the use of mixtures of hydrophilic gels to effect particular release rates. However, the same hydrophilic gel or mixture of hydrophilic gels must be utilized in the core and sustained-release enveloping layers in order that applicant's mathematical expression be useful in determining the appropriate quantity of medicament. It is, of course, possible that by judicious choice of different hydrophilic gels and their mixtures in the core and enveloping layers, a tablet could be prepared providing substantially zero order release of medicament. However, applicant's mathematical expression would not predict the amounts of medicament to be used in the core and layers. Applicant prefers HPMC, particularly HPMC having a viscosity of 4000 mPa.s, as a hydrophilic gel for use in the present invention. Applicant also prefers a mixture of hydrophilic gels containing 1 part of HPMC having a viscosity of 4000 mPa.s and 3 parts of HPMC having a viscosity of 100 mPa.s.

Applicant's tablets can contain one or more excipients in addition to the medicament and hydrophilic gel. With the exception of tablet disintegrants, which tend to break apart the tablet, any other pharmaceutical excipient may be used. Suitable tablet excipients for use in applicant's tablets are, for example, diluents such as lactose, dextrose, mannitol, sorbitol, gelatin, acacia, dicalcium phosphate, tricalcium phosphate or monocalcium phosphate; binders such as sucrose, polyethylene glycol, polyvinylpyrrolidone; lubricants such as stearic acid, zinc, calcium or magnesium stearate, or talc, colorants such as FD&C dyes (i.e. dyes permissible in food, drugs and cosmetics), and flavoring agents. In practicing the present invention, it is apparent that the amount of medicament in the core or a sustained release layer is determined by applicant's formula and the amount of hydrophilic gel is determined by the desired release rate. The amount of pharmaceutical excipients varies as necessary to provide the total weight required for the core or layer. The total weight required is a quantity readily determined by those with ordinary skill in the art and depends on the volume of the core or layer and the compressibility of the materials. In the preferred tablet formulations of this invention, applicant prefers that the diluents, mannitol and lactose, be used to complete the total weight required of the core and layers. Applicant further prefers that the lubricant, zinc stearate, be used in all compressed tablet formulations.

Medicaments which are suitable for use in the solid dosage forms of this invention include any drug substance or combination of drug substances which can be formulated in a solid dosage form and for which sustained release administration would be desirable. Suitable drug substances and their indications are acetaminophen, an antipyretic; aminophylline, a smooth muscle relaxant; amitriptyline HCl, an antidepressant; betamethasone phosphate, a glucocorticoid; brompheniramine maleate, an antihistaminic; buphenine HCl, a peripheral vasodilator; carbetapentane citrate, an antitussive; carbocromene HCl, a coronary vasodilator; chlorpheniramine maleate, an antihistaminic; chlorpromazine HCl, a tranquilizer; clonidine HCl; an antihypertensive; codeine phosphate, an antitussive; diethylpropion HCl, an anorexiant; dihydroergotamine mesylate, a cerebral vasodilator; dextromethorphan HBr, an

3

antitussive; diphylline, a bronchondilator; ergotamine tartrate, a vasoconstrictor; fenfluramine HCl, an anorexiant; ferritin, an antianemic; furosemide, a diuretic antihypertensive; heptaminol HCl, a cardiotonic; hydroquinidine HCl, an antiarrhythmic; ibuprofen, an anti-inflammatory; imipramine HCl, an antidepressant; indomethacin, an anti-inflammatory; isoxsuprine HCl, a vasodilator; isosorbide dinitrate, a coronary vasodilator; metformin HCl, a hypoglycemic agent; melperone, a neuroleptic; methscopalamine bromide, an antispasmodic; noscopine HCl, an antitussive; oxeladin citrate, an antitussive; papaverine HCl, a smooth muscle relaxant and cerebral vasodilator; pentazocine HCl, an analgesic; phenylephrine HCl, a sympathomimetic vasoconstrictor; phenylpropanolamine HCl, a sympathomimetic bronchodilator; potassium chloride, a potassium source for the treatment of hypokalemia; procainamide HCl, an antiarrhythmic; propranolol HCl, a beta receptor blocker, antihypertussive and antiarrhythmic agent; pseudoephedrine HCl, a bronchodilator and peripheral vasoconstrictor; theophylline, a smooth muscle relaxant, bronchodilator and myocardial stimulant; terbutaline sulfate, a bronchodilator; terfenadine, an antihistamine; and trihexyphenidyl HCl, an anti-Parkinsonian agent. Terbutaline, pseudoephedrine HCl, terfenadine, melperone and 4 - ethyl - 1,3 - dihydro - 5 - (4 - pyridinylcarbonyl) - 2$H$ - imidazol - 2 - one are preferred drug substances.

Although any drug substance can be administered by sustained release, for various reasons, certain drug substances when administered via sustained release formulations, offer little or no advantage over more conventional modes of administration. For example, sustained release administration is contraindicated or is of questionable value for a) drugs with long biological half lives (i.e., greater than 10 hours) such as chlorpromazine and thioridazine; b) drugs absorbed by active transport, as for example, quaternary ammonium compounds such as propantheline bromide; c) antibiotics such as penicillins and cephalosporins; d) drugs destroyed by first pass liver metabolism and/or metabolism in the gut wall such as ritodrine, salicylamide or lidocaine; and drugs that are naturally sustained release, for example, medicaments which are absorbed in body fat and subsequently are slowly released into the patients blood, such as chlorpromazine and thioridazine.

Any amount of medicament can be formulated into the solid dosage forms of the present invention. Due to the limitations on the size of the dosage from and the large amount of medicament necessary in the core, dosage forms of this invention should not exceed about 500 mg of medicament. Applicant prefers dosage forms having 300 mg or less of medicament. Dosage forms of this invention having greater than 300 mg of medicament will require departure from cylindrical shapes to oval or capsule shaped forms in order to accommodate the high concentration of medicament in the core of the dosage form. In principle, there is no lower limit on the amount of medicament which can be formulated into the dosage forms of this invention. However, applicant prefers dosage forms having at least 0.1 mg of medicament.

The rate of medicament release from applicant's tablets, whether by tablet erosion or by diffusion, is determined by medicant concentration as well as the hydrophilic gel and its concentration. Other factors, such as compression force in pressed tablets, appear to have little, if any, effect. The relationship between release rate and medicament concentration is linear, that is, as the medicament concentration is doubled, for example, the release rate is likewise doubled. Reducing the hydrophilic gel concentration in a given dosage form, increases the release rate of the medicament in a semi-logarithmic manner. That is, by reducing the concentration of a given hydrophilic gel by half in an otherwise identical dosage form, the release rate of the medicament would increase by a factor of approximately 1.25. Thus, it is apparent that by judicious choice of medicament concentration, hydrophilic gel concentration and the choice of hydrophilic gel, a wide range of release rates can readily be obtained.

The sustained dosage forms of applicant's invention are intended to effect substantially zero order release of medicament from 1 hour to 2 days, preferably from 4 to 24 hours, more preferably from 8 to 12 hours depending on medicament concentration as well as the hydrophilic gel and its concentration. In order to assure zero order release of medicament, the concentration of hydrophilic gel in the core and sustained-release, enveloping layers must be essentially constant.

The solid dosage forms of this invention can be of any shape, however, in using applicant's mathematical expression, it will be necessary to orient, at least conceptually, the shape of the dosage form to correspond to a cylinder and designate lines thru the center of the dosage form corresponding to the cylindrical axis of height and radial axis. For example, in the typical biconvex circular tablet, the axis of height extends from the center of one convex surface to the other through the center of the tablet. The radial axis extends outward from the axis of height at the tablets center to the circular edge. In solid dosage forms having a capsule shape, the axis of height and radial axis are readily apparent by analogy to a cylindrical solid.

In determining the amount of medicament to be used in the core or any layer, it is necessary to choose a radial axis along which the differential radius of the core or a layer is measured. Where the shape of the solid dosage form is such that the axis corresponding to the radial axis of a cylinder is not readily apparent, some trial and error may be necessary.

To determine the amount of medicament to be used in the core or in any layer, the applicant has derived a mathematical expression based on certain theoretical considerations. Although the derivation of this expression was based on solid dosage forms having the shape of a cylindrical solid and solid dosage forms which release medicament by erosion, the resulting expression has been found to be useful in

4

# 0 111 144

dosage forms of a variety of shapes and dosage forms which release medication by diffusion as well as erosion.

As used herein, the differential radius of a given dosage form is the thickness of the core or a given layer measured along the radial axis. Thus for the core, the differential radius is the distance from the center of the tablet to the outer edge of the core measured along the radial axis. The differential radius of a given layer is the distance from the inner edge to the outer edge of a given layer measured along the radial axis. Where the tablet shape is such that the measurement along the radial axis will vary depending on the direction of the axis, an average value for differential radius should be used. For example, in tablets which are prismoidal solids, the length of the radial axis will vary depending on whether the axis extends outward toward a corner of the tablet edge, toward a face of the tablet edge or toward a point in between.

To determine the amount of medicament to be used in the core or an enveloping layer of the solid dosage forms of applicant's invention, the following mathematical expression has been derived:

$$D_L = D_T \times \frac{R_D}{R_T}$$

wherein $D_L$ is the amount of medicament in the core or in any given layer, $D_T$ is the total amount of medicament in the sustained-release portion of the tablet, $R_D$ is the differential radius for the core or the enveloping layer for which the amount of required medicament is to be determined and $R_T$ is the radius of the sustained-release portion of the tablet.

Although the above expression has general application to solid dosage forms which release medicament by erosion and diffusion, applicant has derived a second expression for dosage forms which release medicament exclusively, or nearly so, by diffusion. The diffusion equation is:

$$D_L = D_T \times \frac{R_{SD}}{R_T^2}$$

wherein $D_L$, $D_T$ and $R_T$ are as in the erosion expression above and wherein $R_{SD}$ is the squared differential radius. The squared differential radius for a given sustained release layer or the core is the difference between the square of the distance from the outer surface of the sustained-release portion of the tablet to the inner edge of the layer to be determined (the center of the tablet for the core) and the square of the distance from the outer surface of the sustained-release portion of the tablet to the outer edge of the layer to be determined (zero for the outermost layer) measured along the radial axis of the tablet. Average values should be used for irregularly shaped dosage forms. Although the latter expression theoretically works better for diffusing dosage forms and thus constitutes a preferred embodiment of this invention, the applicant nevertheless prefers the former erosion expression for use in calculating doses for all solid dosage forms of this invention.

A tablet of this invention wherein the distribution of medicament in the core and sustained release layers is determined by means of the above-mentioned mathematical expression, will release medication in a substantially zero-order fashion. That is, the amount of medicament released from a tablet of applicant's invention per unit of time will remain essentially constant until all medicament is released. While the release is normally substantially constant, in some instances there may be an initial release of medicament in a first order fashion, that is, initially more medicament will be released per unit of time than will be released during a subsequent unit of time which more closely imitates the desired zero-order release. The excess amount of medication released is said to be "dumped". This dumping effect occurs prior to and during the time period in which the tablet's hydrophilic gel layers are swelling to form a barrier to retard further release of medicament. Normally, this dumping period will not exceed a few hours, generally it does not exceed about 1 hour.

Where it is desirable to reduce or eliminate this dumping effect, it will be necessary to deviate from the medicament distribution pattern predicted by applicant's mathematical expression. Because the dumped medication originates from the outermost sustained release layer, the typically, substantially zero-order release of medicament achieved by applicant's tablets can approximate constant release even more closely and any initial dumping of medicament reduced to negligible amounts, by transferring some of the medication from the outermost sustained release layer to the inner layers or core. Preferably, this is accomplished by transferring an amount of medicament from the outermost sustained release layer to the penultimate sustained release layer or in the case of a tablet having only 1 sustained release layer, transferring some of the medicament from the sustained release layer to the core.

The amount of medicament to be transferred from the outermost sustained release layer in order to reduce the dumping effect is approximately equal to the amount of medicament "dumped". Because all of the medicament dumped is assumed to come from the outermost sustained release layer in the first hour, the amount of medicament dumped is approximately equal to the difference between the amount of medicament released in the first hour and the amount released in any subsequent hour.

The following examples illustrate the use of the above-mentioned mathematical expression in preparing various solid dosage forms.

5

Example 1
Sample calculation for cylindrical dosage form having two enveloping layers

|                  | Active ingredient | 50 mg |            |
| ---------------- | ----------------- | ----- | ---------- |
|                  | Differential radii |      |            |
| Core             |                   | 6.35 mm | (1/4 inch) |
| Middle layer     |                   | 6.35 mm | (1/4 inch) |
| Outer layer      |                   | 12.70 mm | (1/2 inch) |

Therefore, $R_T$=25.40 mm (1 inch)

Using the erosion expression for the core:

$$D_L = 50 \text{ mg} \times \frac{6.35}{25.4} = 12.5 \text{ mg}$$

for the middle layer:

$$D_L = 50 \text{ mg} \times \frac{6.35}{25.4} = 12.5 \text{ mg}$$

for the outer layer:

$$D_L = 50 \text{ mg} \times \frac{12.7}{25.4} = 25 \text{ mg}$$

Example 2
Sample calculation for capsule shaped tablet having two enveloping layers

The tablet has a core wherein the axis of length is 14.3 mm long and the radial axis measures 2.8 mm. The middle layer has an axis of length equal to 16.7 mm and the distance from the tablets center to the outer edge of the layer measured along the radial axis is 4 mm. The outer layer has an axis of length equal to 19 mm and the distance from the tablets center to the outer edge measured along the radial axis is 5.2 mm. The total amount of medicament is equal to 500 mg. The total radius measured along the radial axis is 5.2 mm.

Using the erosion expression for the core:
the differential radius is equal to the length of the core's radial axis, 2.8 mm.

$$D_L = 500 \text{ mg} \times \frac{2.8}{5.2} = 270 \text{ mg}$$

for the middle layer:
the differential radius is the difference between the length of the radial axis of the middle layer and the radial axis of the core, 1.2 mm

$$D_L = 500 \text{ mg} \times \frac{1.2}{5.2} = 115 \text{ mg}$$

for the outer layer:
the differential radius is the difference between the length of the radial axes of the outer and inner layers, 1.2 mm.

$$D_L = 500 \text{ mg} \times \frac{1.2}{5.2} = 115 \text{ mg}$$

Example 3
Sustained release tablet of 1-(4-fluorophenyl)-4-(4-methyl-1-piperidinyl)-1-butanone hydrochloride having two enveloping layers

Tablet specifications

| | |
|---|---|
| Total active ingredient | 50 mg |
| Hydrophilic gel | 18% HPMC 4000 mPa.s |
| Lubricant | 1.5% zinc stearate |
| Filler | mannitol (to weight required) |

Differential radii

| | | |
|---|---|---|
| Core | 6.35 mm | (1/4 inch) |
| Middle layer | 2.38 mm | (3/32 inch) |
| Outer layer | 2.38 mm | (3/32 inch) |

Therefore, using the erosion expression:

Distribution of active ingredient

| | |
|---|---|
| Core | 28.6 mg |
| Middle layer | 10.7 mg |
| Outer layer | 10.7 mg |

Therefore, suitable formulations are:

| | Core (mg) | Middle layer (mg) | Outer layer (mg) |
|---|---|---|---|
| 1-(4-Fluorophenyl)-4-(4-methyl-1-piperidinyl)-1-butanone hydrochloride | 28.6 | 10.7 | 10.7 |
| Mannitol | 51.9 | 134.2 | 190.55 |
| HPMC 4000 mPa.s | 18.0 | 32.4 | 45.0 |
| Zinc stearate | 1.5 | 2.7 | 3.75 |
| Total Weight Requirement | 100 mg | 180 mg | 250 mg |

Example 4
Capsule shaped tablet of melperone having two enveloping layers

Tablet specifications

| | |
|---|---|
| Active Melperone | 50 mg |
| Hydrophilic gel | HPMC 4000 mPa.s 18%/layer |
| Lubricant | zinc stearate 1.5%/layer |
| Filler | lactose (to weight required) |

Differential radii

| | | |
|---|---|---|
| Core | 2.41 mm | (0.095 inch) |
| Middle layer | 0.64 mm | (0.025 inch) |
| Outer layer | 0.95 mm | (0.0375 inch) |
| Total Radius | 4.00 mm | (0.1575 inch) |

7

Therefore the granulations required are:

|  | Core (mg) | Middle layer (mg) | Outer layer (mg) |
|---|---|---|---|
| Melperone | 30 | 8 | 12 |
| HPMC | 12 | 22 | 52 |
| Zinc stearate | 1 | 1.8 | 4.4 |
| Lactose | 25 | 88.2 | 221.6 |
| Total Weight Requirement | 65 mg | 120 mg | 290 mg |

Example 5
Melperone extruded suppository having two enveloping layers

Specifications

| Active Melperone | 50 mg/dosage form |
|---|---|
| Hydrophilic gel | HPMC D-50 40%/layer |
| Excipients | Glycerin 10% of gel weight |
| Water | to weight required |

Differential radii

| Core | 3 mm |
|---|---|
| Middle layer | 3 mm |
| Outer layer | 3 mm |

Therefore, the required Melperone is as follows:

| Core | 16.67 mg |
|---|---|
| Middle layer | 16.67 mg |
| Outer layer | 16.67 mg |

Therefore, the required formulations are as follows:

|  | Core (mg) | Middle layer (mg) | Outer layer (mg) |
|---|---|---|---|
| Melperone | 16.67 | 16.67 | 16.67 |
| HPMC E-50 | 60.0 | 296.0 | 444.0 |
| Glycerin | 6.0 | 29.6 | 44.4 |
| Water | 67.33 | 397.73 | 604.93 |
| Total Weight Requirement | 150 mg | 740 mg | 1110 mg |

8

# 0 111 144

Example 6
Terbutaline sulfate buccal tablet formulation

### Tablet specification

| | |
|---|---|
| Terbutaline Sulfate | 2 mg/tablet |
| Hydrophilic gel | HPMC 4000 mPa.s 18%/layer |
| Lubricant | zinc stearate 1.5%/layer |
| Filler | mannitol (to weight required) |

### Differential radii

| | | |
|---|---|---|
| Core | 1.6 mm | (1/16 inch) |
| Middle layer | 0.8 mm | (1/32 inch) |
| Outer layer | 0.8 mm | (1/32 inch) |

Therefore, the required formulations are:

| | Core (mg) | Middle layer (mg) | Outer layer (mg) |
|---|---|---|---|
| Terbutaline sulfate | 1.0 | 0.5 | 0.5 |
| HPMC 4000 mPa.s | 2.7 | 5.4 | 9.9 |
| Zinc stearate | 0.225 | 0.45 | 0.825 |
| Mannitol | 11.075 | 23.65 | 43.775 |
| Total Weight Requirement | 15.0 mg | 30.0 mg | 55.0 mg |

Example 7
Sample calculation for tablet with two enveloping layers using the diffusion expression

### Tablet specifications

| | |
|---|---|
| Active ingredient | 50 mg |
| Total Radius | 25.40 mm (1 inch) |

### Differential radii

| | | |
|---|---|---|
| Core | 6.35 mm | (1/4 inch) |
| Middle layer | 6.35 mm | (1/4 inch) |
| Outer layer | 12.70 mm | (1/2 inch) |

Squared differential radii
For the core:
    measured along the radial axis, the distance from the outer surface to the tablet center is 25.40 mm (1 inch) and the distance from the outer surface to the outer edge at the core is 19 mm (3/4 inch). Therefore,
$$R_{SD}=(25.40)^2-(19)^2=284.16 \text{ mm}^2$$
(in inches: $R_{SD}=(1)^2-(3/4)^2=7/16 \text{ inch}^2$)

Similarly, for the middle layer:
$$R_{SD}=(19)^2-(12.70)^2=199.71$$
(in inches: $R_{SD}=(3/4)^2-(1/2)^2=5/16$)

for the outer layer:
$$R_{SD}=(12.70)^2-0=161.29$$
(in inches: $R_{SD}=(1/2)^2-0=1/4$)

9

0 111 144

Therefore, the distribution of active ingredient is calculated as follows:
for the core:

$$D_L = 50 \times \frac{284.16}{(25.40)^2} = 22.022 \text{ mg}$$

$$(\text{in inches: } D_L = 50 \times \frac{7/16}{1} = 21.875 \text{ mg})$$

for the inner layer:

$$D_L = 50 \times \frac{199.71}{(25.40)^2} = 15.48 \text{ mg}$$

$$(\text{in inches: } D_L = 50 \times \frac{5/16}{1} = 15.625 \text{ mg})$$

for the outer layer:

$$D_L = 50 \times \frac{161.29}{(25.40)^2} = 12.5 \text{ mg}$$

$$(\text{in inches: } D_L = 50 \times \frac{1/4}{1} = 12.5 \text{ mg})$$

**Claims for the Contracting States: BE, CH, DE, FR, GB, IT, LI, LU, NL, SE**

1. A pharmaceutical tablet providing substantially zero-order release of medicament having a core and one or more enveloping sustained-release layers, the core and sustained-release enveloping layers consisting essentially of a mixture containing medicament, hydrophilic gel and excipients, wherein the concentration of hydrophilic gel in the core and in each sustained-release enveloping layer is essentially constant and wherein the amount of medicament in either the core or in any given sustained-release layer is determined by the formula

$$D_L = D_T \times \frac{R_D}{R_T}$$

wherein $D_L$ is the amount of medicament in the core or in any given sustained-release layer, $D_T$ is the total amount of medicament in the sustained-release portion of the tablet, $R_D$ is the differential radius for the core or the enveloping layer for which the amount of required medicament is to be determined and $R_T$ is the radius of the sustained-release portion of the tablet.

2. A tablet of claim 1 having one enveloping layer.

3. A tablet of claim 2 wherein the hydrophilic gel is hydroxypropyl methylcellulose.

4. A tablet of claim 3 wherein the viscosity of the hydroxypropyl methylcellulose is 4000 mPa.s.

5. A tablet of claim 2 wherein the medicament is terbutaline.

6. A tablet of claim 2 wherein the medicament is terfenadine.

7. A tablet of claim 2 wherein the medicament is pseudoephedrine hydrochloride.

8. A tablet of claim 2 having an outer enveloping non-sustained, release layer.

9. A tablet of claim 8 wherein the medicament in the outer non-sustained, release layer is terfenadine and the medicament in the enveloping, sustained-release layer and core is pseudoephedrine hydrochloride.

10. A tablet of claim 1 having from 2 to 4 enveloping layers.

11. A tablet of Claim 10 wherein the hydrophilic gel is hydroxypropyl methylcellulose.

12. A tablet of claim 11 wherein the viscosity of the hydroxypropyl methylcellulose is 4000 mPa.s.

13. A tablet of claim 10 wherein the medicament is terbutaline.

14. A tablet of claim 10 wherein the medicament is terfenadine.

15. A tablet of claim 10 wherein the medicament is pseudoephedrine hydrochloride.

16. A tablet of claim 1 having 2 enveloping, sustained-release layers.

17. A tablet of claim 16 having an outer enveloping non-sustained, release layer.

18. A tablet of claim 17 wherein the medicament in the outer non-sustained, release layer is terfenadine and the medicament in the enveloping, sustained-release layers and core is pseudoephedrine hydrochloride.

10

## 0 111 144

**Claims for the Contracting State: AT**

1. A process for preparing a pharmaceutical tablet providing substantially zero-order release of medicament having a core and one or more enveloping sustained-release layers, the core and sustained-release enveloping layers consisting essentially of a mixture containing medicament, hydrophilic gel and excipients, wherein the formulation is effected in such a way that the concentration of hydrophilic gel in the core and in each sustained-release enveloping layer is essentially constant and wherein the amount of medicament which is added in either the core or in any given sustained-release layer is to be determined by the formula

$$D_L = D_T \times \frac{R_D}{R_T}$$

wherein $D_L$ is the amount of medicament provided for the core or for any given sustained-release layer, $D_T$ is the total amount of medicament provided for the sustained-release portion of the tablet, $R_D$ is the differential radius for the core or the enveloping layer for which the amount of required medicament is to be determined and $R_T$ is the radius of the sustained-release portion of the tablet.

2. A process according to claim 1, wherein the tablet has one enveloping layer.

3. A process according to claim 2 wherein the hydrophilic gel is hydroxypropyl methylcellulose.

4. A process according to claim 3 wherein the viscosity of the hydroxypropyl methylcellulose is 4000 mPa.s.

5. A process according to claim 2 wherein the medicament is terbutaline.

6. A process according to claim 2 wherein the medicament is terfenadine.

7. A process according to claim 2 wherein the medicament is pseudoephedrine hydrochloride.

8. A process according to claim 2 wherein the tablet has an outer enveloping non-sustained, release layer.

9. A process according to claim 8 wherein the medicament in the outer non-sustained, release layer is terfenadine and the medicament in the enveloping, sustained-release layer and core is pseudoephedrine hydrochloride.

10. A process according to claim 1 wherein the tablet has from 2 to 4 enveloping layers.

11. A process according to claim 10 wherein the hydrophilic gel is hydroxypropyl methylcellulose.

12. A process according to claim 11 wherein the viscosity of the hydroxypropyl methylcellulose is 4000 mPa.s.

13. A process according to claim 10 wherein the medicament is terbutaline.

14. A process according to claim 10 wherein the medicament is terfenadine.

15. A process according to claim 10 wherein the medicament in the tablet is pseudoephedrine hydrochloride.

16. A process according to claim 1 wherein the tablet has two enveloping, sustained-release layers.

17. A process according to claim 16 wherein the tablet has an outer enveloping non-sustained, release layer.

18. A process according to claim 17 wherein the medicament in the tablet in the outer non-sustained, release layer is terfenadine and the medicament in the enveloping, sustained-release layers and core is pseudoephedrine hydrochloride.

**Patentansprüche für die Vertragsstaaten: BE, CH, DE, FR, GB, IT, LI, LU, NL, SE**

1. Pharmazeutische Tablette, dadurch gekennzeichnet, daß die Freisetzung des Arzneistoffes im wesentlichen nach nullter Ordnung erfolgt und die Tablette einen Kern und mindestens eine Hüllschicht mit verzögerter Wirkungsabgabe aufweist, wobei der Kern und die Hüllschichten mit verzögerter Wirkungsabgabe im wesentlichen aus einem Gemisch von Arzneistoff, hydrophilem Gel und Trägern bestehen, wobei die Konzentration des hydrophilen Gels im Kern und in jeder Hüllschicht mit verzögerter Wirkungsabgabe im wesentlichen konstant ist und wobei die Menge des Arzneistoffes entweder im Kern oder in jeder der vorliegenden Hüllschichten mit verzögerter Wirkungsabgabe durch die folgende Gleichung bestimmt ist

$$D_L = D_T \times \frac{R_D}{R_T}$$

in der $D_L$ die Menge des Arzneistoffes im Kern oder in jeder der vorliegenden Hüllschichten mit verzögerter Wirkungsabgabe bedeutet, $D_T$ die Gesamtmenge des Arzneistoffes im Tablettenanteil mit verzögerter Wirkungsabgabe darstellt, $R_D$ den charakteristischen Radius des Kerns oder der Hüllschicht, für die die Menge des erforderlichen Arzneistoffes bestimmt werden soll, bedeutet, und $R_T$ den Radius des Tablettenanteils mit verzögerter Wirkungsabgabe darstellt.

11

2. Tablette nach Anspruch 1, mit einer Hüllschicht.

3. Tablette nach Anspruch 2, in der das hydrophile Gel Hydroxypropylmethylcellulose ist.

4. Tablette nach Anspruch 3, in der die Viskosität von Hydroxypropylmethylcellulose 4000 mPa.s beträgt.

5. Tablette nach Anspruch 2, in der der Arzneistoff Terbutalin ist.

6. Tablette nach Anspruch 2, in der der Arzneistoff Terfenadin ist.

7. Tablette nach Anspruch 2, in der der Arzneistoff Pseudoephedrin-hydrochlorid ist.

8. Tablette nach Anspruch 2, mit einer äußeren Hüllschicht ohne verzögerte Wirkungsabgabe.

9. Tablette nach Anspruch 8, in der der Arzneistoff in der äußeren Hüllschicht ohne verzögerte Wirkungsabgabe Terfenadin ist und der Arzneistoff in der Hüllschicht mit verzögerter Wirkungsabgabe und im Kern Pseudoephedrin-hydrochlorid ist.

10. Tablette nach Anspruch 1, mit zwei bis vier Hüllschichten.

11. Tablette nach Anspruch 10, in der das hydrophile Gel Hydroxypropylmethylcellulose ist.

12. Tablette nach Anspruch 11, in der die Viskosität von Hydroxypropylmethylcellulose 4000 mPa.s beträgt.

13. Tablette nach Anspruch 10, in der der Arzneistoff Terbutalin ist.

14. Tablette nach Anspruch 10, in der der Arzneistoff Terfenadin ist.

15. Tablette nach Anspruch 10, in der der Arzneistoff Pseudoephedrin-hydrochlorid ist.

16. Tablette nach Anspruch 1, mit zwei Hüllschichten mit verzögerter Wirkungsabgabe.

17. Tablette nach Anspruch 16, mit einer äußeren Hüllschicht ohne verzögerte Wirkungsabgabe.

18. Tablette nach Anspruch 17, in der der Arzneistoff in der äußeren Hüllschicht ohne verzögerte Wirkungsabgabe Terfenadin ist und der Arzneistoff in den Hüllschichten mit verzögerter Wirkungsabgabe und im Kern Pseudoephedrin-hydrochlorid ist.

**Patentansprüche für den Vertragsstaat: AT**

1. Verfahren zur Herstellung einer pharmazeutischen Tablette, bei der die Freizetzung des Arzneistoffes im wesentlichen nach nullter Ordnung erfolgt und die Tablette einen Kern und mindestens eine Hüllschicht mit verzögerter Wirkungsabgabe aufweist, wobei der Kern und die Hüllschichten mit verzögerter Wirkungsabgabe im wesentlichen aus einem Gemisch von Arzneistoff, hydrophilem Gel und Trägern bestehen, wobei die Konfektionierung so erfolgt, daß die Konzentration des hydrophilen Gels im Kern und in jeder Hüllschicht mit verzögerter Wirkungsabgabe im wesentlichen konstant ist und wobei die Menge des entweder im Kern oder in jeder der vorliegenden Hüllschichten mit verzögerter Wirkungsabgabe zugesetzten Arzneistoffes durch die folgende Gleichung bestimmt ist:

$$D_L = D_T \times \frac{R_D}{R_T}$$

in der $D_L$ die Menge des für den Kern oder für jede der vorliegenden Hüllschichten mit verzögerter Wirkungsabgabe verwendeten Arzneistoffs bedeutet, $D_T$ die Gesamtmenge des für den Tablettenanteil mit verzögerter Wirkungsabgabe verwendeten Arzneistoffs darstellt, $R_D$ den charakteristischen Radius des Kerns oder der Hüllschicht, für die die Menge des erforderlichen Arzneistoffes zu bestimmen ist, bedeutet, und $R_T$ den Radius des Tablettenanteils mit verzögerter Wirkungsabgabe darstellt.

2. Verfahren nach Anspruch 1, in dem die Tablette eine Hüllschicht aufweist.

3. Verfahren nach Anspruch 2, in dem das hydrophile Gel Hydroxypropylmethylcellulose ist.

4. Verfahren nach Anspruch 3, in dem die Viskosität von Hydroxypropylmethylcellulose 4000 mPa.s beträgt.

5. Verfahren nach Anspruch 2, in dem der Arzneistoff Terbutalin ist.

6. Verfahren nach Anspruch 2, in dem der Arzneistoff Terfenadin ist.

7. Verfahren nach Anspruch 2, in dem der Arzneistoff Pseudoephedrin-hydrochlorid ist.

8. Verfahren nach Anspruch 2, in dem die Tablette eine äußere Hüllschicht ohne verzögerte Wirkungsabgabe aufweist.

9. Verfahren nach Anspruch 8, in dem der Arzneistoff in der äußeren Hüllschicht ohne verzögerte Wirkungsabgabe Terfenadin ist und der Arzneistoff in der Hüllschicht mit verzögerter Wirkungsabgabe und im Kern Pseudoephedrin-hydrochlorid ist.

10. Verfahren nach Anspruch 1, in dem die Tablette 2 bis 4 Hüllschichten aufweist.

11. Verfahren nach Anspruch 10, in dem das hydrophile Gel Hydroxypropylmethylcellulose ist.

12. Verfahren nach Anspruch 11, in dem die Viskosität von Hydroxypropylmethylcellulose 4000 mPa.s beträgt.

13. Verfahren nach Anspruch 10, in dem der Arzneistoff Terbutalin ist.

14. Verfahren nach Anspruch 10, in dem der Arzneistoff Terfenadin ist.

15. Verfahren nach Anspruch 10, in dem der Arzneistoff in der Tablette Pseudoephedrin-hydrochlorid ist.

16. Verfahren nach Anspruch 1, in dem die Tablette 2 Hüllschichten mit verzögerter Wirkungsabgabe aufweist.

17. Verfahren nach Anspruch 16, in dem die Tablette eine äußere Hüllschicht ohne verzögerte Wirkungsabgabe aufweist.

18. Verfahren nach Anspruch 17, in dem der Arzneistoff in der Tablette in der äußeren Hüllschicht ohne verzögerte Wirkungsabgabe Terfenadin ist und der Arzneistoff in den Hüllschichten mit verzögerter Wirkungsabgabe und im Kern Pseudoephedrin-hydrochlorid ist.

## Revendications pour les Etats Contractants: BE, CH, DE, FR, GB, IT, LI, LU, NL, SE

1. Un comprimé pharmaceutique assurant une libération essentiellement d'ordre zéro d'un médicament, ayant un noyau et une ou plusieurs couches d'enveloppe à libération contrôlée, le noyau et les couches d'enveloppe à libération contrôlée consistant essentiellement en un mélange contenant un médicament, un gel hydrophile et des excipients où la concentration du gel hydrophile dans le noyau et dans chaque couche d'enveloppe à libération contrôlée est essentiellement constante et où la quantité de médicament dans le noyau ou l'une quelconque donnée des couches à libération contrôlée est déterminée par la formule

$$D_L = D_T \times \frac{R_D}{R_T}$$

dans laquelle $D_L$ est la quantité de médicament dans le noyau ou dans l'une quelconque donnée des couches à libération contrôlée, $D_T$ est la quantité totale de médicament dans la portion à libération contrôlée du comprimé, $R_D$ est le rayon différentiel du noyau ou de la couche d'enveloppe dont on détermine la quantité de médicament requise et $R_T$ est le rayon de la portion à libération contrôlée du comprimé.

2. Un comprimé selon la revendication 1 ayant une couche d'enveloppe.

3. Un comprimé selon la revendication 2, dans lequel le gel hydrophile est de l'hydroxypropylméthyl-cellulose.

4. Un comprimé selon la revendication 3, dans lequel la viscosité de l'hydroxypropylméthylcellulose est de 4 000 mPa.s.

5. Un comprimé selon la revendication 2, dans lequel le médicament est la terbutaline.

6. Un comprimé selon la revendication 2, dans lequel le médicament est la terfénadine.

7. Un comprimé selon la revendication 2, dans lequel le médicament est le chlorhydrate de pseudo-éphédrine.

8. Un comprimé selon la revendication 2 ayant une couche d'enveloppe extérieure qui n'est pas à libération contrôlée.

9. Un comprimé selon la revendication 8, dans lequel le médicament dans la couche extérieure qui n'est pas à libération contrôlée est la terfénadine et le médicament dans la couche d'enveloppe à libération contrôlée et le noyau est le chlorhydrate de pseudo-éphédrine.

10. Un comprimé selon la revendication 1 ayant 2 à 4 couches d'enveloppe.

11. Un comprimé selon la revendication 10, dans lequel le gel hydrophile est l'hydroxypropylméthyl-cellulose.

12. Un comprime selon la revendication 11, dans lequel la viscosité de l'hydroxypropylméthylcellulose est de 4 000 mPa.s.

13. Un comprimé selon la revendication 10, dans lequel le médicament est la terbutaline.

14. Un comprimé selon la revendication 10, dans lequel le médicament est la terfénadine.

15. Un comprimé selon la revendication 10, dans lequel le médicament est le chlorhydrate de pseudo-éphédrine.

16. Un comprimé selon la revendication 1 ayant 2 couches d'enveloppe à libération contrôlée.

17. Un comprimé selon la revendication 16 ayant une couche extérieure d'enveloppe qui n'est pas à libération contrôlée.

18. Un comprimé selon la revendication 17, dans lequel le médicament dans la couche extérieure qui n'est pas à libération contrôlée est la terfénadine et le médicament dans les couches d'enveloppe à libération contrôlée et le noyau est le chlorhydrate de pseudo-éphédrine.

## Revendications pour l'Etat Contractant: AT

1. Un procédé pour la préparation d'un comprimé pharmaceutique assurant une libération essentiellement d'ordre zéro d'un médicament, ayant un noyau et une ou plusieurs couches d'enveloppe à libération contrôlée, le noyau et les couches d'enveloppe à libération contrôlée consistant essentiellement en un mélange contenant un médicament, un gel hydrophile et des excipients, où la formulation est effectuée de telle sorte que la concentration du gel hydrophile dans le noyau et dans chaque couche d'enveloppe à libération contrôlée soit essentiellement constante et où la quantité de médicament que l'on

13

ajoute dans le noyau ou l'une quelconque des couches à libération contrôlée donnée est déterminée par la formule

$$D_L = D_T \times \frac{R_D}{R_T}$$

dans laquelle $D_L$ est la quantité de médicament dans le noyau ou dans l'une quelconque des couches à libération contrôlée donnée, $D_T$ est la quantité totale de médicament dans la portion à libération contrôlée du comprimé, $R_D$ est le rayon différentiel du noyau ou de la couche d'enveloppe dont on détermine la quantité de médicament requise et $R_T$ est le rayon de la portion à libération contrôlée du comprimé.

2. Un procédé selon la revendication 1 ayant une couche d'enveloppe.

3. Un procédé selon la revendication 2, dans lequel le gel hydrophile est l'hydroxypropylméthyl-cellulose.

4. Un procédé selon la revendication 3, dans lequel la viscosité de l'hydroxypropylméthylcellulose est de 4 000 mPa.s.

5. Un procédé selon la revendication 2, dans lequel le médicament est la terbutaline.

6. Un procédé selon la revendication 2, dans lequel le médicament est la terfénadine.

7. Un procédé selon la revendication 2, dans lequel le médicament est le chlorhydrate de pseudo-éphédrine.

8. Un procédé selon la revendication 2, dans lequel le comprimé a une couche d'enveloppe extérieure qui n'est pas à libération contrôlée.

9. Un procédé selon la revendication 8, dans lequel le médicament dans la couche extérieure qui n'est pas à libération contrôlée est la terfénadine et le médicament dans la couche d'enveloppe à libération contrôlée et le noyau est le chlorhydrate de pseudo-éphédrine.

10. Un procédé selon la revendication 1, dans lequel le comprimé a 2 à 4 couches d'enveloppe.

11. Un procédé selon la revendication 10, dans lequel le gel hydrophile est l'hydroxypropylméthyl-cellulose.

12. Un procédé selon la revendication 11, dans lequel la viscosité de l'hydroxypropylméthylcellulose est de 4 000 mPa.s.

13. Un procédé selon la revendication 10, dans lequel le médicament est la terbutaline.

14. Un procédé selon la revendication 10, dans lequel le médicament est la terfénadine.

15. Un procédé selon la revendication 10, dans lequel le médicament dans le comprimé est le chlorhydrate de pseudo-éphédrine.

16. Un procédé selon la revendication 1, dans lequel le comprimé a 2 couches d'enveloppe à libération contrôlée.

17. Un procédé selon la revendication 16, dans lequel le comprimé a une couche extérieure d'enveloppe qui n'est pas à libération contrôlée.

18. Un procédé selon la revendication 17, dans lequel le médicament dans le comprimé dans la couche extérieure qui n'est pas à libération contrôlée est la terfénadine et le médicament dans les couches d'enveloppe à libération contrôlée et le noyau est le chlorhydrate de pseudo-éphédrine.